# EUROPEAN PATENT APPLICATION

(11) **EP 1 736 142 A1**
(43) Date of publication of application: **27.12.2006**
(21) Application number: 05450023.6
(22) Date of filing: 03.02.2005
(51) Int. Cl.: A61K 8/97, A61K 8/37, A61K 8/368, A61Q 19/08, A61Q 19/02, A61Q 17/04, A61Q 5/02

(54) **Anti-photoaging composition comprising mulberry extract**

(71) Applicant: Luanratana, Omboon, Bangkok 10400 (TH)
(72) Inventor: Luanratana, Omboon, Bangkok 10400 (TH)
(74) Representative: Patentanwaltskanzlei Matschnig

(57) **Abstract**

Mulberry extract may be provided in cosmeceutical raw material forms, including a paste, liquid or powder that can be added to cosmetics to provide whitening effects for darkened skin through anti-tyrosinase activity, and antioxidant and anti-inflammatory properties.

## Description

### RELATED APPLICATION

This application claims the priority of Thai Application No. 088455, filed on February 4, 2004 in the name of Omboon Luanratana.

### BACKGROUND

The youth and beauty of skin depends on skin radiance without freckles. Exposure to UV-radiation from sunlight induces the darkening of skin, which is called tanned skin if the dark color is evenly distributed, or freckles when unevenly distributed or over-produced. Melanin is the skin pigment that gives the skin its color. Greater amounts of melanin result in darker skin. The synthesis of melanin is a normal biological activity in human, animal, plant and microorganisms. The synthesis of melanin involves transformation of tyrosine into Dopa or 3,4-dihydroxyphenylalanine by the enzyme tyrosinase, which is then transformed into quinone compounds and dopachrome, which will be further oxidized by other enzymes giving melanin pigment. Tyrosinase is found in melanocytes, the amount of which depends on genetic build-up. Since the oxidation of the amino acid tyrosine is the rate-limiting step in melanin production in humans and animals, skin color depends on the activity of tyrosinase enzyme, in addition to other genetic factors.

Exposure to UV-radiation and free radicals stimulates the enzyme, resulting in a darkening of the skin. Moreover, sunlight also causes inflammation of the skin, causing tiny spots which, at the very least, results in uneven coloring of the skin. This effect is clearly seen on people's faces, creating beauty and personality problems. Over-reactions can lead to skin cancer. In addition to this, free radicals in the environment and from UV-radiation are also the cause of premature aging, which is obvious on the face and uncovered skin.

Scientists accept that free radicals are the cause of cell deterioration, aging, or premature aging. The formation of lipofuschin or aging pigment is the result of exposure to sunlight. This can be seen in elderly skin due to chronic accumulation. The formation of dark pigment also involves free radicals, therefore the compounds having antioxidant properties help reduce aging and the formation of dark spots.

Pharmaceutical and cosmetic industries have been searching for compounds having anti-tyrosinase activity. The pure compounds that are normally used are arbutin, kojic acid, and hydroquinone. The first two compounds have given good results in in-vitro testing but have been clinically ineffective. Moreover, the use of hydroquinone is restricted to prescription by a medical doctor. The side effects of long term use of hydroquinone include permanent freckles or chloasma.

The cortex mori extract and the ramulus mori extract are used but not as widely as the above chemicals. It has been reported that the young branches of *Morus alba* contain the anti-tyrosinase activity and also inhibit the synthesis of melanin pigment by melanoma-B16. This extract, having oxyresveratrol as the active compound, also reduced the melanin formation on guinea pig's skin that was exposed to UV radiation without causing any irritation or allergic reaction.

Only small amounts of Mulberry extract have been added to cosmetics due to the low percentage of dried Mulberry residue in the available commercial Mulberry extracts and the high price of the extracts. Present cosmetics using Mulberry extracts were limited to sunscreen products and nourishing day and night creams with small quantities of Mulberry extract. Present Mulberry extracts do not show either anti-inflammatory activity or antioxidant characteristics. For example, see U.S. Pat. Nos. 6,827,944; 6,338,855 and 6,280,715, which note that Mulberry extracts are useful as tyrosinase inhibitors and as whitening agents, but do not indicate that they are used as anti-inflammatory or antioxidant agents.

### SUMMARY

In one embodiment, a process is provided for obtaining Mulberry extract that is of higher quality than presently available commercial extracts. This process allows the industrial production of Mulberry cosmeceuticals in a shorter time.

In another embodiment, Mulberry extracts are provided in cosmeceutical raw material forms, including a paste, liquid or powder that can be added to cosmetics. These cosmeceuitcal raw materials have concentrations of Mulberry extract ranging from 0.1 %-100%.

The Mulberry extracts and/or cosmeceutical raw materials may be used in medicine and food supplements, and cosmetics for the face, body, scalp, and hair in the form of cream, lotion, serum or other forms of liquid, pressed facial powder, cover stick, lipstick and lip gloss in the form of stick, tube, or other containers, by mixing with other plant extracts and other compounds used in cosmetics. These cosmetics provide lightening effects for the darkened skin through tyrosinase activity, antioxidant, anti-inflammatory and antiallergy, which are also the causes of darkened skin in humans. The overall effect is skin nourishment and rejuvenation. These cosmetics are capable of lightening the skin after sun exposure and reducing photoaging.

This invention provides in several various embodiments cosmeceutical compositions containig Mulberry extracts obtained from the wood and bark of Mulberry branches and roots. There is scientific evidence that these extracts inhibit melanin formation by anti-tyrosinase activity and free radical scavenger properties, and inhibit cyclo-oxygenase activity, the enzyme which is responsible for inflammation. These properties make possible anti-inflammatory activity and recovery of prior skin condition after UV exposure.

### BRIEF DESCRIPTION OF THE DRAWINGS

The accompanying drawings illustrate various embodiments of the present invention and are a part of the specification. The illustrated embodiments are merely examples of the present invention and do not limit the scope of the invention.

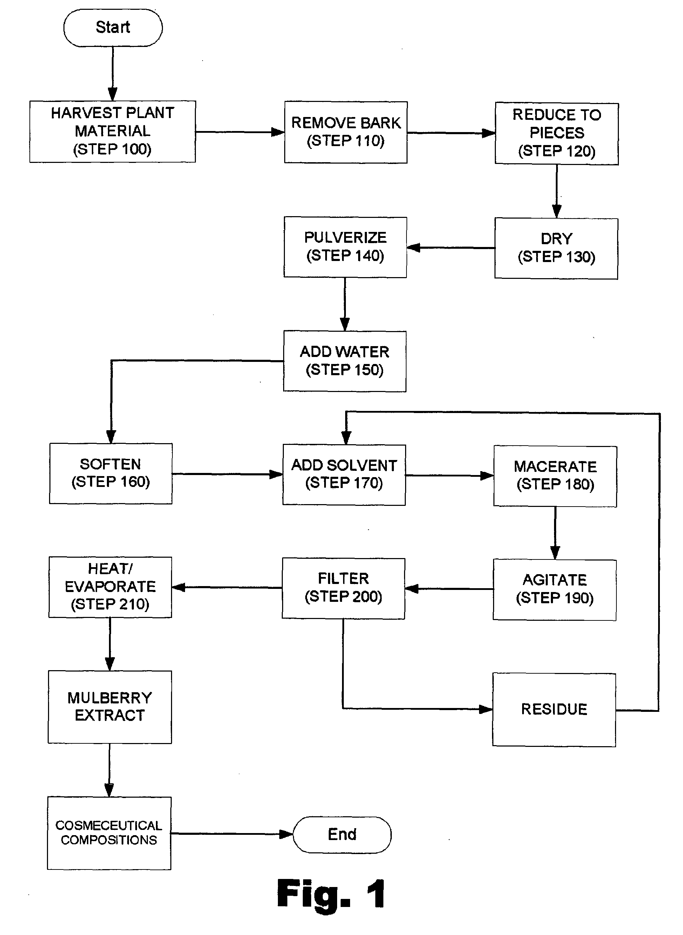

Figure 1 is a flow chart illustrating one embodiment of a process of producing Mulberry extract.

Figure 2 is a chart showing the inhibition of tyrosinase activity in various concentrations of Mulberry extract from the wood of the branches.

Figure 3 is a chart showing the inhibition of tyrosinase activity in various concentrations of Mulberry extract from the wood of the roots.

Figure 4 is a chart showing the inhibition of tyrosinase activity in various concentrations of Mulberry extract from the bark of the root.

Figure 5 is thin layer chromatogram showing the antioxidant properties of Mulberry extract.

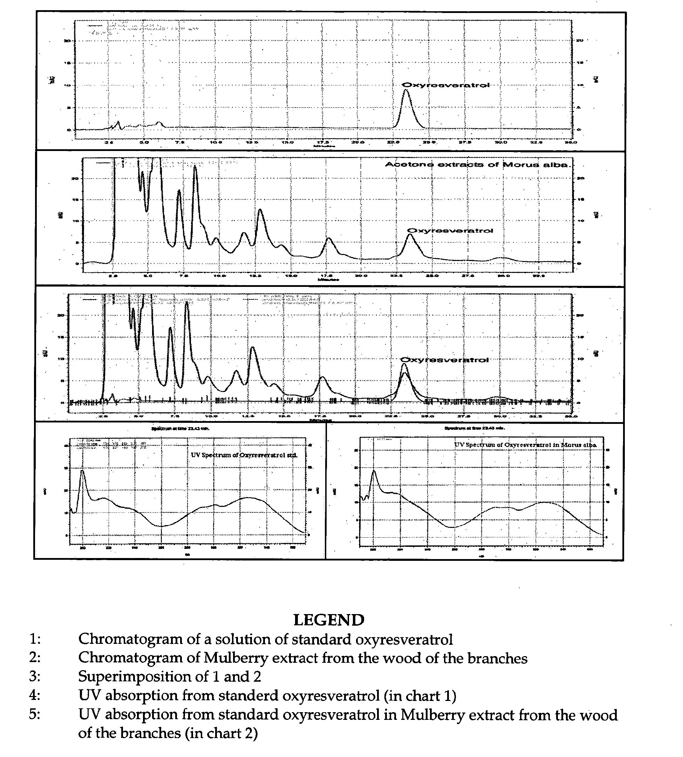

Figure 6 is an HPLC chromatogram showing the presence of an active substance having anti-tyrosinase, antioxidant, and anti-inflammatory properties.

Throughout the drawings, identical reference numbers designate similar, but not necessarily identical, elements.

### DETAILED DESCRIPTION

The present invention offers a better process of Mulberry extract production in a shorter time with a higher percentage of dried residue. Also included in the invention are the compositions of Mulberry cosmeceutical ingredients to be added to various cosmetics. Some of these cosmeceutical ingredients are off-white, brownish yellow, or pale brown in color and can be mixed well in cosmetic formulas without creating unwanted brown coloration.

The Mulberry cosmeceuticals of this invention contain stilbene compounds, resveratrol, and oxyresveratrol, which exhibit anti-tyrosinase activity and antioxidant properties. The extracts also contain flavonoids, which have antioxidant properties, and other compounds that exhibit anti-inflammatory activity by inhibiting cyclo-oxygenase 1 and cyclo-oxygenase 2. The cosmetics contained in this present invention can, therefore, whiten the skin, restore skin conditions after sun exposure, and reduce premature aging, especially photoaging.

The cosmetics contain about 0.01 % to about 10% Mulberry extract, and are typically medicine and food supplements, cosmetics for the face, body, scalp, and hair in the form of cream, lotion, serum or other forms of liquid, pressed facial powder, cover stick, lipstick and lip gloss in the form of stick, tube, or other containers. These cosmetics may also be mixed with other plant extracts and other compounds used in cosmetics.

Mulberry extracts may be taken from Mulberry plants which are grown in Thailand and are of native plants in Thailand such as *Morus rotundiloba* Koidz, *Morus laevigata* Wall; or are hybrids obtained from cross between the former two plants and *Morus alba* Linne which are widely distributed in Thailand including the sericulture cultivars Nakornrajasima 60, Burirum 60, Burirum 51 and Srisaket 33.These cosmetics contain the cosmeceutical raw material from the extract of the Thai Mulberry plant, wood and bark of the branches and roots .

According to the present invention, industrial scale preparation of cosmeceuticals from the wood and bark of the branches and roots of Mulberry plants are of higher quality than the presently available commercial Mulberry extract. The improved process for obtaining Mulberry extract includes harvesting Mulberry branches with a diameter ranging from about 1 cm to about 2.5 cm (step 100). Preferably, the bark is then removed immediately after harvesting, although it may be removed within about 24 hours of harvesting (step 110). The bark is removed, preferably with a knife or other sharp edge, from the lower end to the top end of the branch, exposing and off white to yellowish white material. The material is then reduced in size to pieces up to about 5 mm thickness, such as by chopping (step 120). These pieces are then dried in the sun or in a hot air oven at a temperature up to about 80°C, preferably less than about 60°C (step 130). The dried chips are then pulverized, such as by a mechanical shredder or grinder, to create a raw material powder (step 140). When stored, the raw material powder is preferably kept in an airtight plastic bag at about room temperature.

The extract is then prepared by adding water containing 0.1 1 %-2% weight by weight (w/w) of Tween 20, 1-5 times w/w of the raw material powder, to the powder and mixing well (step 150). The mixture is then allowed to stand for about 30 minutes, or until the cell walls of the plant material have softened (step 160). An organic solvent is then added in an amount of about 6-20 times w/w of the raw material powder (step 170). Suitable solvents include, but are not limited to, alcohol, glycols, acetone, chloroform, or ethyl acetate. The mixture is then macerated for about 1 to about 3 hours (step 180), and then agitated at a speed of about 60 to about 120 rpm at a temperature ranging from about 20°C to about 60°C for about 20 minutes to about 6 hours (step 190). The mixture is then filtered (step 200), and the residue can be reused for additional extractions, following the same process just described. The filtrates are then combined and heated to evaporate off all the organic solvent and the aqueous phase, leaving the Mulberry extract (step 210). The heating preferably is done under reduced pressure at a temperature not exceeding about 80°C, preferably about 60°C.

The cosmeceutical raw material, herein referred to as Mulberry Cosmeceutical 1, contains Mulberry extract may be provided in three different forms: a paste, a liquid, and a powder. The paste is made by adding to about 60% to about 80% Mulberry extract about 20% to about 40% w/w organic solvent such as ethyl alcohol, glycerine, sorbitol, propylene glycol or butylene glycol, with about 0.1 % to about 2% w/w of a preservative such as methyl paraben, propyl paraben, sodium benzoate or benzoic acid. The organic solvent may be just one solvent, or it may be a combination of 2-3 solvents. The preservative may also be just one of the above preservatives, or it may be of a mixture of 2-3 preservatives.

The liquid form is made by adding to the extract an inorganic solvent at a concentration ranging from about 80%-90% w/w inorganic solvent. Suitable inorganic solvents include, but are not limited to, purified water, buffered water, or aromatic water. The inorganic solvent may be added in combination with up to about 5% w/w organic solvents. Suitable organic solvents include, but are not limited to, ethyl alcohol, glycerine, sorbitol, propylene glycol, or butylene glycol. A suspending agent is also added at a concentration of about 0.2% to about 2% w/w, such as sorbitan sesquioleate, carbopol, emulgin, ceteareth-25, or a mixture of 2-3 suspending agents. Preservatives such as methyl paraben, propyl paraben, sodium benzoate or benzoic acid or a mixture of 2-3 preservatives are also added, thus resulting in a liquid suspension.

The powder form can be prepared by adding 5%-50% w/w of lactose, maltodextrin, zinc oxide, talcum, or corn starch to the liquid cosmeceutical raw material.

The Mulberry Cosmeceutical 1 in the form of a sticky mass, suspension, or dried powder in certain cases is not suitable for use in cosmetics. However, it may be formulated into an appropriate form, herein referred to as Mulberry Cosmeceutical 2, having the following composition: about 1% to about 60% w/w of the cosmeceutical raw material; about 5% to about 25% w/w of inorganic solvent such as purified water, aqueous buffer, or aromatic water; about 10% to about 35% w/w of organic solvent such as ethyl alcohol, glycerine, sorbitol, propylene glycol, or butylene glycol; about 1% to about 2% w/w of suspending agents such as sorbitan sesquioleate, carbopol, emulgin, or ceteareth-25 or a combination of these suspending agents; about 0.5% to about 2% w/w of preservative such as methylparaben, propylparaben, sodium benzoate, or benzoic acid or the combination of these preservatives; about 0% to about 2% w/w anti-oxidants such as vitamin C, ascorbic palmitate, tocoferol, or vitamin E acetate or its combination; and up to about 2% w/w fragrance.

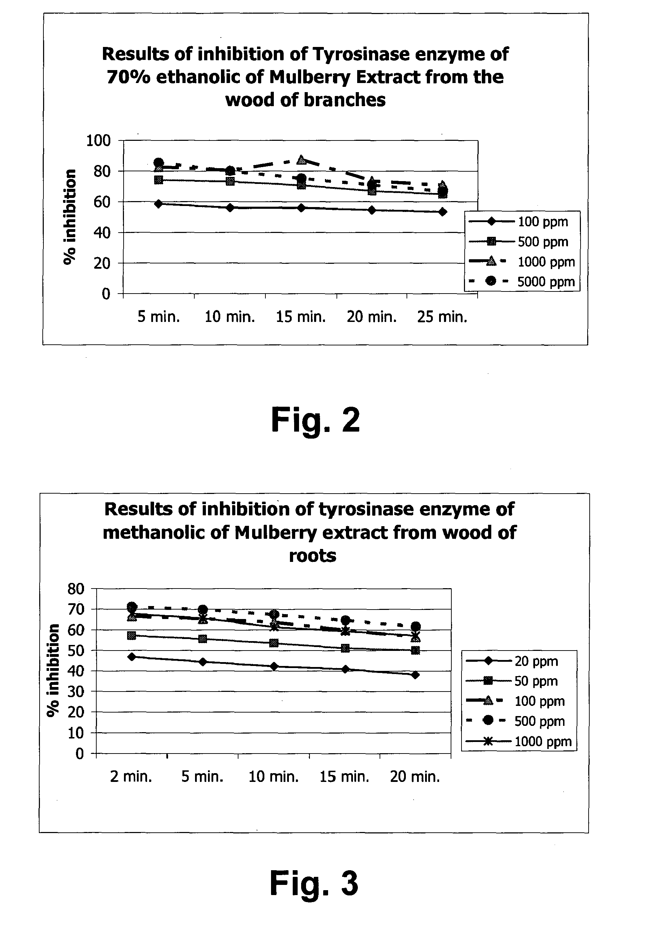

The inhibition of tyrosinase enzyme activity of Mulberry Cosmeceutical 1 was measured, the results being shown in Figures 2-4. The inhibition of tyrosinase enzyme activity can be measured by mixing the desired extract with the enzyme and the substrate (L-Dopa). If the tested extract contains tyrosinase activity, the color in the test tube will be paler than the control tube which contains only the enzyme and the substrate. This brown coloration can be measured at 475 nm, therefore the percentage of inhibition can be measured.

Figure 2 shows more than 50% inhibition of tyrosinase activity of the extract from the wood of the Mulberry branches at a concentration of 100 ppm. The concentration 500-5,000 ppm gives 60% inhibition.

Figure 3 shows 50%-60% inhibition of tyrosinase activity of the extract from the wood of the Mulberry roots at a concentration of 50-1,000 ppm. The concentration 20 ppm gives 40% inhibition.

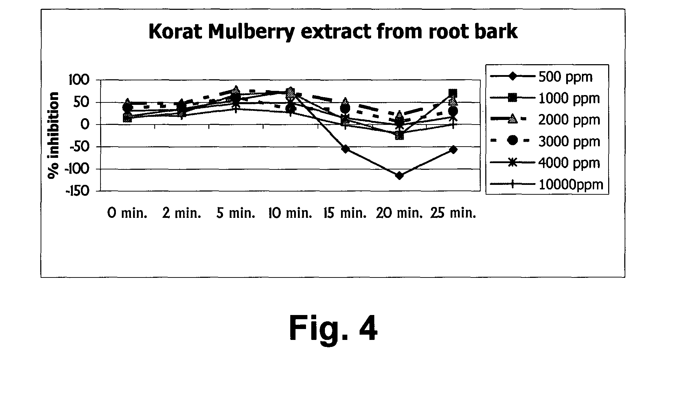

Figure 4 shows 50% inhibition of tyrosinase activity of the extract from the bark of the Mulberry roots at a concentration of 2,000-4,000 ppm. Higher or lower concentration does not give inhibition.

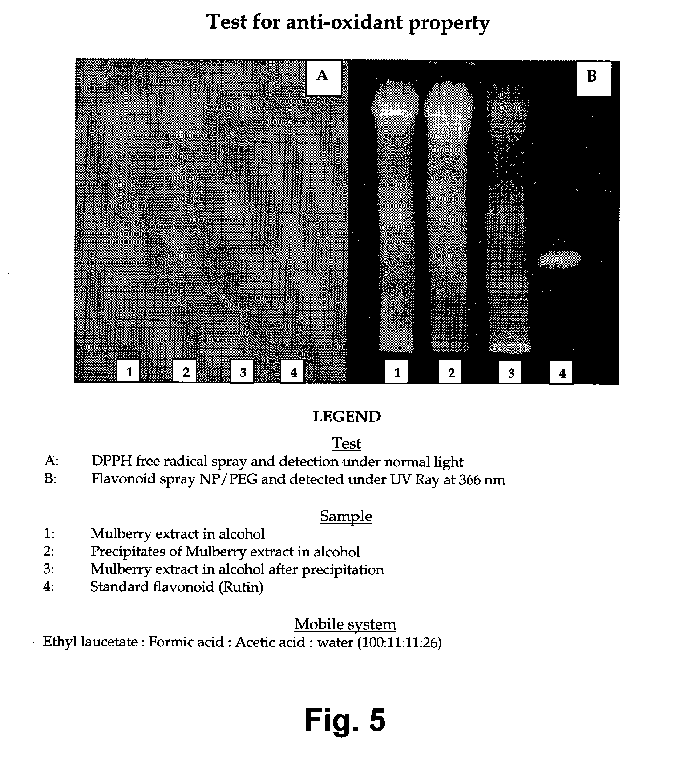

The extracts were also tested for antioxidant properties. One can test for antioxidant properties by using Thin Layer Chromatography (TLC) and detecting with DPPH free radical spray, which has a magenta color. If the tested compounds contain the antioxidant property they will appear as yellow to white spots on the magenta background. Figure 5 shows the Mulberry extract containing various components having free radical scavenging properties, which can be seen as the yellow or white bands on the TLC chromatogram. The precipitate, which is normally filtered off through the aging process of conventional extraction processes, also showed antioxidant properties. For this reason, the cosmeceutical raw material of this invention possesses high antioxidant activity because the precipitate was not removed.

The test for anti-inflammatory activity can be done on lab mice by applying ethyl phenyl propyolate on mice ears at a concentration of 1 mg per ear to induce inflammation. The fullest swollen ear can be observed 1 hour after the application, then the tested extracts and reference compound, dexamethasone, are applied onto the inflamed ears at a dose of 0.05 mg/ear. The reduction in the thickness of the ears in comparison with the control group indicates anti-inflammatory activity, as shown in Table 1.

**Table 1 Percentage reduction of inflamed ears of experimental mice after application of Mulberry extracts**

| **Experimental Group** | **0 min** | **60 min** | **90 min** | **120 min** | **240 min** | **360 min** | **480 min** |
|---|---|---|---|---|---|---|---|
| | | | | | | | |
| Control* | 0 | 56.427 | 60.302 | 57.372 | 54.442 | 51.229 | |
| 47.543 | | | | | | | |
| Anti-inflammatory** | 0 | 54.253 | 42.533 | 38.752 | 25.142 | 13.422 | |
| 11.531 | | | | | | | |
| Wood, Branch | 0 | 53.403 | 25.425 | 20.699 | 14.556 | 12.287 | |
| 10.019 | | | | | | | |
| Bark, Branch | 0 | 58.034 | 33.932 | 44.896 | 27.694 | 29.112 | |
| 29.938 | | | | | | | |
| Bark, root | 0 | 60.019 | 39.792 | 33.176 | 31.758 | 28.072 | |
| 25.520 | | | | | | | |
| Wood, root | 0 | 63.043 | 45.274 | 39.480 | 38.280 | 32.514 | |
| 33.365 | | | | | | | |

| | | | | | | | |
|---|---|---|---|---|---|---|---|
| *Control = the mice received neither anti-inflammatory agents nor Mulberry extracts ** Anti-inflammatory = Dexamethasone | | | | | | | |

**,** The results in Table 2 show that when neither anti-inflammatory agents nor Mulberry extracts were used, as in the control group, the recovery of the inflammation was very slow. When the anti-inflammatory agent was used the recovery was faster. When the extracts from the wood and bark of the branches and roots were used as the anti-inflammatory agents, it was found that the recovery from inflammation also increased with time. The extract from the wood of the branches had the strongest anti-inflammatory activity.

The enzymes involved in the inflammation process are cyclo-oxygenase -1 (COX-1) and cyclo-oxygenase -2 (COX-2), which synthesizes the postaglandin that induces inflammation from the biological substance in humans and animals. When the activity of these enzymes is inhibited, the inflammatory reaction does not occur. The inhibited activity of COX-1 and COX-2 can be evaluated with a commercial kit. The results are read with an ELISA reader (Table 2).

**Table 2: Percentage Inhibition of Cyclo-oxygenase (COX-1), (COX-2)**

| **Tested Extract** | **Concentration mg/ml** | **% Inhibition COX-1** | **% Inhibition COX -2** |
|---|---|---|---|
| **Wood, Branches** | 0.05 | 89.20 | 61.95 |
| **Wood, Roots** | 1.0 | 61.25 | 40.55 |
| **Bark, Branches** | 0.7 | 63.05 | 77.10 |
| **Bark, Roots** | 1.0 | 89.95 | 8.40 |

From the results obtained in Table 2, it can be concluded that all the tested Mulberry extracts inhibit the activity of the enzymes involved in the inflammatory process, cyclo-oxygenase 1 and 2. The extract from the wood of the Mulberry branches contained the highest activity, which can be observed from less concentration required but higher percentage of inhibition, more than 60%. The second potent extract is the bark of the branches, which gave the same inhibition effect but at 14 times the concentration of the wood. The extract from the bark and wood of the roots also inhibited the activity of both enzymes but at 20 times concentration of the wood. It was also less effective on COX-2.

The Mulberry extracts and/or cosmeceutical raw materials may be used in medicine and food supplements, and cosmetics for the face, body, scalp, and hair in the form of cream, lotion, serum or other forms of liquid, pressed facial powder, cover stick, lipstick and lip gloss in the form of stick, tube, or other containers, by mixing with other plant extracts and other compounds used in cosmetics. These cosmetics include cleansing cream, bar and liquid soap, moisturizer, after sun cream, sun-screen, mask, eye cream or gel, night cream, lip gloss and lipstick, shampoo and cream rinse, shaving cream, aftershave lotion, or antiperspirant cream or spray in solid or liquid form.

The above cosmetics can also include other medicinal plant extracts such as *Clitorea ternatea, Citrus hystrix, Tamarindus indicus, Aloe vera, Clerodendrum inerme, Sapindus emarginatus, Sapindus trifoliatus, Pueraria mirifica, Glycine max, Vigna radiata,Sesamum indicum, Passiflora foetida, Mirabilis jalapa, Ardisia elliptica, Garcinia mangostana, Curcuma longa,Zingiber cassumunar, Curcuma xanthorrhiza*, *Stephania venosa, Gynura pseudochina, Centella asiatica, Phyllanthus emblica, Sapindus rarak, Eclipta protata, E. alba, Zingiber officinale, Hydnocarpus anthelminthicus, Zollingeria dongnaiensis, Lawsonia inermis, Cymbopogon citratus, Rhinacanthus nasutus, Andrographis paniculata, Tinospora crispa, Acacia concinna, Averrhoa carambola, Chromolaena odoratum, Pogostemon auricularis, Collagen,* tea tree oil.

The cosmetics may also include any of the compounds shown below in Table 3.

| | |
|---|---|
| Skin conditioner | Dialkyldimethylpolysiloxane, Dimethicone, Polyoxyethylene, and Cationiccellulose ether |
| Surface active agent | Ammoniumlauryl sulfate and Sodium lauryl ether sulfate |
| Hair conditioner | Betaine |
| Preservative | Methylparaben, Propylparaben, Butylparaben, Sodium benzoate, Ascorbic acid, BHT, Tocopherol, and Vitamin E acetate |
| Moisturizer | Squalene, Soy bean oil, Lanolin alcohol, Dimethicone, Olive oil, Jojoba oil, Rice germ oil, Avocado oil, Coconut oil, Mung bean oil, Stearyl alcohol, Myristyl myristate, Glycerylmonostearate, Aloe vera, Collagen, Plancental extract, Cutina-MD, Mineral oil, Allantoin, Glycerine, and Sorbitol |
| Hardening agent | Stearic acid, Cetylyl alcohol, Stearyl alcohol, and White Bee wax |
| Deodorant | Aluminum chlorohydroxide, Sodium aluminum chlorhydrolactate, Magnesium aluminium silicate, and Aluminum chlorohydrate |
| Suspending agent | Sodiumcarboxylmethylcellulose, Crystallinecarboxymethylcellulose |
| Body builder | Bentonite and Hydroxyethylcellulose |
| Emulsifier | Glyceryl monostearate, Sorbitan sesquioleate, Laureth-23, Steareth-6, Leximul 561, Lecithin, Xanthan gum, Sodiumlaurate ethersulfate, Polyoxyethylene fatty ester, TWEEN-20, TWEEN-40, TWEEN-60, TWEEN-80, and Phosphate ester |
| Sunscreen | Benzophenone-2, Benzophenone-4, Teona-G, Titanium dioxide, Zinc oxide, Methyl anthranilate, Octyl salicylate, Octyl dimethyl PABA, and Octylmethoxylcinnamate |
| Masking agent | Titanium dioxide, Zinc oxide, Talcum, Clay, Tanaka, and Kaolin |
| Solvent | Water, Propylene glycol, Butylene glycol, Glycerine, Sorbitol, and Ethyl alcohol |
| Alkali | Sodium hydroxide, Sodium citrate, and Triethanolamine |
| Buffer | Citric acid and sodium citrate |
| Antioxidant | Vitamin E acetate and Sodium ethylene diamine trichloroacetic acid |
| Fat | Lanolin, White Bee Wax, Wool Fat, Soybean oil, Mung bean oil, Rice bran oil, Sesame oil, Avocado oil, and spermaceti |

### Bar and Liquid Soap

Bar soap contains general ingredients such as fat (plant or animal origin) and alkali. The production method dissolves alkali in an appropriate amount of water, dissolves fat by heating and reducing the temperature to that of the alkaline solution and adding the alkaline solution into the liquefied fat, stirring slowly. At this stage, add about 0.01 % to about 5% w/w of Mulberry Cosmeceutical 1 or Mulberry Cosmeceutical 2and add fragrance as required. The resulting bar soap will have the properties of the Mulberry extract: whitening, anti-oxidant, and anti-inflammation.

Glycerine bar soap contains glycerine and anionic or non-ionic surfactants, solvent, skin conditioner, emulsifier and preservative. This can be mixed prior to use or bought in ready made, solid form. The solid is liquefied at a temperature not more than about 80°C. At this stage, add about 0.01 % to about 5% w/w of Mulberry Cosmeceutical 1 or Mulberry Cosmeceutical 2 and add fragrance as required. The resulting bar soap will have the properties of the Mulberry extract: whitening, anti-oxidant, and anti-inflammation.

Liquid soap contains anionic or non-ionic surfactant, skin conditioner, emulsifier, pearlescent, and preservative, which can be mixed prior to use or bought as a ready made liquid. Add about 0.01 % to about 5% w/w of Mulberry Cosmeceutical 1 or Mulberry Cosmeceutical 2 and add fragrance as required. The resulting liquid soap will have the properties of the Mulberry extract: whitening, anti-oxidant, and anti-inflammation.

Additionally, the above cosmetics may contain about 0.5% to about 5.0% w/w of the following plant extracts: *Pueraria mirifica, Sesamum indicum, Aloe vera, Clerodendrum inerme, Passiflora foetida, Mirabilis jalapa, Ardisia elliptica, Garcinia mangostana, Curcuma longa, Zingiber cassumunar, Curcuma xanthorrhiza, Stephania venosa, Gynura pseudochina, Centella asiatica,* or *Phyllanthus emblica.*

### Cream or Lotion

The general composition of the cream base is synthetic fat or natural fat (plant or animal in origin); emulsifier; mineral oil, vegetable oil, or synthetic oil; distilled or purified water; preservatives; fragrance agent; and colorant. Also included is skin softener, surfactant, moisturizer, cleansing agent, sunscreen, or skin nutrients to suit the needs of the cream or lotion. These include cleansing cream and lotion, moisturizing cream and lotion, eye gel or cream, night cream and lotion, and sunscreen cream and lotion. At the stage before adding the required fragrance and color, add about 0.01 % to about 10% w/w of Mulberry Cosmeceutical 1 or Mulberry Cosmeceutical 2. The resulting cream, lotion, or gel will have the properties of the Mulberry extract: whitening, anti-oxidant, and anti-inflammation resulting in lighter skin color and prevention of premature aging.

Additionally, the above cosmetics may contain about 0.5% to about 5.0% w/w of the following plant extracts: *Pueraria mirifica, Glycine max, Vigna radiata, Sesamum indicum, Aloe vera, Clerodendrum inerme, Passiflora foetida, Mirabilis jalapa, Ardisia elliptica, Garcinia mangostana, Curcuma longa, Zingiber cassumunar, Curcuma xanthorrhiza, Stephania venosa, Gynura pseudochina, Centella asiatica, Phyllanthus emblica, Chromolaena odoratum, Pogostemon auricularis,* or tea tree oil.

### After Sun Cream and Lotion

The general composition is as above with the addition of about 0.01 % to about 1% w/w *Curcuma longa* extract, about 0.1 % to about 10% w/w soya bean extract, and about 0.1% to about 10% w/w *Aloe vera* extract. At the stage before adding the required fragrance and color, add about 0.01 % to about 10% w/w of Mulberry Cosmeceutical 1 or Mulberry Cosmeceutical 2. The resulting cream or lotion will have the properties of the Mulberry extract, whitening, anti-oxidant, and anti-inflammation resulting in lighter skin color and a reduced burning sensation.

Additionally, the above cosmetics may contain about 0.5% to about 5.0% w/w of the following plant extracts: *Vigna radiata, Sesamum indicum, Aloe vera, Mirabilis jalapa, Ardisia elliptica, Curcuma longa, Zingiber cassumunar, Curcuma xanthorrhiza, Stephania venosa, Gynura pseudochina,* or *Centella asiatica.*

### Anti-wrinkle Cream for Face, Neck and Under Chin

The general composition is as above with the addition of about 0.1 % to about 10% w/w of *Pueraria mirifica,* about 0.01 % to about 1% w/w of *Curcuma longa* extract, about 0.1 % to about 10% w/w of soya bean extract, about 0.1 % to about 10% w/w of *Aloe vera* extract, and about 0.01 % to about 2% w/w of collagen. At the stage before adding the required fragrance and color, add about 0.01 % to about 10% w/w of Mulberry Cosmeceutical 1 or Mulberry Cosmeceutical 2. The resulting cream or lotion will have the properties of the Mulberry extract: whitening, anti-oxidant, and anti-inflammation resulting in lighter skin and reduced wrinkles on the face, neck, and under chin.

Additionally, the above cosmetics may contain about 0.5% to about 5.0% w/w of the following plant extracts; *Pueraria mirifica, Glycine max, Vigna radiata, Sesamum indicum, Aloe vera, Mirabilis jalapa, Curcuma longa, Zingiber cassumunar, Curcuma xanthorrhiza, Stephania venosa, Gynura pseudochina, or Centella asiatica.*

### Face Mask Cream

The general composition is as above with the addition of about 5% to about 20% w/w of UV reflectants such as titanium dioxide, zinc oxide, clay, or talcum and other plant extracts such as about 0.1 % to about 10% w/w of *Pueraria miirifica,* about 0.01 % to about 1% w/w of *Curcuma longa* extract, about 0.1% to about 10% w/w of soya bean extract, and about 0.1 % to about 10% w/w of *Aloe vera* extract. At the stage before adding the required fragrance and color, add about 0.01% to about 10% w/w of Mulberry Cosmeceutical 1 or Mulberry Cosmeceutical 2. The resulting cream or lotion will have the properties of the Mulberry extract: whitening, anti-oxidant, and anti-inflammation resulting in lighter skin and when used under make-up it helps make-up stay fresh looking all day.

Additionally, the above cosmetics may contain about 0.5% to about 5.0% w/w of the following plant extracts: *Pueraria mirifica, Glycine max, Vigna radiata, Sesamum indicum, Aloe vera, Clerodendrum inerme, Passiflora foetida, Mirabilis jalapa, Ardisia elliptica, Garcinia mangostana, Curcuma longa, Zingiber cassumunar, Curcuma xanthorrhiza, Stephania venosa, Gynura pseudochina, Centella asiatica, Chromolaena odoratum, Pogostemon auricularis,* or tea tree oil.

### Lip Gloss and Lipstick

The general composition is about 20% to about 40% w/w vegetable fat and/or animal fat, about 20% to about 50% w/w of mineral oil or solvent, about 5% to about 10% w/w solidifier, up to about 20% w/w of colorants and fragrance, and about 10% to about 20% w/w moisturizer. At the stage before adding the required fragrance and color, add about 0.01% to about 10% w/w of Mulberry Cosmeceutical 1 or Mulberry Cosmeceutical 2. The resulting lip-gloss and lipstick will have the properties of the Mulberry extract: whitening, anti-oxidant, and anti-inflammation resulting in lighter lip skin color.

Additionally, the above cosmetics may contain about 0.5% to about 5.0% w/w of the following plant extracts: *Pueraria mirifica, Glycine max, Vigna radiata, Sesamum indicum, Aloe vera, Clerodendrum inerme, Passiflora foetida, Mirabilis jalapa, Ardisia elliptica, Garcinia mangostana, Curcuma longa, Zingiber cassumunar, Curcuma xanthorrhiza, Stephania venosa, Gynura pseudochina, Centella asiatica, Phyllanthus*

### emblica, Chromolaena odoratum, Pogostemon auricularis, or tea tree oil.

### Shampoo and Cream Rinse

The general composition is about 40% to about 50% w/w of anionic or non-ionic surfactant or both, about 1% to about 5% w/w of foaming agent, about 1% to about 5% w/w of emulsifier, about 1% to about 5% w/w of hair conditioner, buffer, preservative, colorants and fragrance, and other plant extracts such as about 10% to about 30% w/w of *Aloe vera, Sapindus emarginatus, S. trifoliatus, Curcuma longa, Clitoria ternatea, Clerodendrum inerme, Ardisia elliptica,* or *Zingiber cassumunar.* At the stage before adding the required fragrance and color, add about 0.01 % to about 10% w/w of Mulberry Cosmeceutical 1 or Mulberry Cosmeceutical 2. The resulting shampoo and cream rinse will have the properties of the Mulberry extract: anti-oxidant and anti-inflammation resulting in a healthy scalp and reduced hair loss.

Additionally, the above cosmetics may contain about 0.5% to about 5.0% w/w of the following plant extracts: tea tree oil, *Eclipta protata, E. alba, Zingiber officinale, Hydnocarpus anthelminthicus, Zollingeria dongnaiensis, Lawsonia inermis, Cymbopogon citratus, Citrus hystrix, Rhinacanthus nasutus, Andrographis paniculata, Tinospora crispa, Acacia concinna, Curcuma longa, Zingiber cassumunar, Clerodendrum inerme, Passiflora foetida,* or *Averrhoa carambola.*

### Stick or Spray Deodorant Cream

The general composition is about 10% to about 25% w/w aluminum chlorhydroxide, about 30% to about 50% w/w of solvent, about 6% to about 10% w/w solidifier, preservatives, colorants and fragrance, and about 1 % to about 5% w/w of other plant extracts such as glycyrrhiza, curcuma longa, or tamarind. At the stage before adding the required fragrance and color, add about 0.01 % to about 10% w/w of Mulberry Cosmeceutical 1 or Mulberry Cosmeceutical 2. The resulting stick or spray deodorant cream will have the properties of the Mulberry extract: whitening, anti-oxidant, and anti-inflammation resulting in lighter colored under arm skin and reduced odor.

Additionally, the above cosmetics may contain about 0.5% to about 5.0% w/w of the following plant extracts: *Sesamum indicum, Aloe vera, Clerodendrum inerme, Passiflora foetida, Mirabilis jalapa, Ardisia elliptica, Garcinia mangostana, Curcuma longa, Zingiber cassumunar, Curcuma xanthorrhiza, Stephania venosa, Gynura pseudochina, Centella asiatica, Chromolaena odoratum, Pogostemon auricularis,* or tea tree oil.

### Anti-inflammatory and Anti-itching Medicine

The addition of about 10% to about 20% w/w of Mulberry Cosmeceutical 1 or Mulberry Cosmeceutical 2 into pharmaceutic aids gives tablet, capsules, liquid dosage forms, suspension, injection, and external applications help in reducing inflammation of the skin, hands, and toe joints.

### Supplementary Food

Other than combining in cosmetics and drugs, the addition of about 5% to about 10% w/w of Mulberry Cosmeceutical 1 or Mulberry Cosmeceutical 2 can be used as ingredients in supplementary food for the antioxidant and anti-inflammatory effects.

Additionally, the above food supplements may contain about 0.5% to about 5.0% w/w of the following plant extracts: *Pueraria mirifica, Glycine max, Vigna radiata, Sesamum indicum, Aloe vera, Curcuma longa, Zingiber cassumunar, Curcuma xanthorrhiza, Stephania venosa, Centella asiatica, Phyllanthus emblica, Zingiber officinale, Cymbopogon citratus, Citrus hystrix, Andrographis paniculata, Tinospora crispa,* or *Averrhoa carambola.*

The preceding description has been presented only to illustrate and describe embodiments of the invention. It is not intended to be exhaustive or to limit the invention to any precise form disclosed. Many modifications and variations are possible in light of the above teaching.

## Claims

1. A cosmeceutical composition, comprising:
from about 60% to about 80% w/w Mulberry extract;
from about 0.1% to about 2% w/w preservatives; and a solvent.

2. The composition of claim 1, wherein said solvent comprises from about 20% to about 40% w/w organic solvent.

3. The composition of claim 1, further comprising:
from about 0.2% to about 2% w/w suspending agent;
wherein said solvent comprises from about 80% to about 90% w/w inorganic solvent and up to about 5% organic solvents.

4. The composition of claim 3, wherein said inorganic solvent comprises purified water, buffered water, or aromatic water.

5. The composition of claim 3, further comprising up to about 50% w/w of lactose, maltodrexrin, zinc oxide, talcum or corn starch.

6. The composition of claim 1, further comprising:
from about 5% to about 25% w/w inorganic solvent;
from about 10% to about 35% w/w organic solvent;
from about 1% to about 2% w/w suspending agent;
up to about 2% w/w anti-oxidant;
from about 0.5% to about 2% w/w preservatives; and
up to about 2% w/w fragrance;
wherein said composition comprises from about 1% to about 60% w/w said Mulberry extract and solvent composition.

7. The composition of claim 1, wherein said composition is included in a cosmetic.

8. The composition of claim 7, wherein said cosmetic includes bar soap, liquid soap, cream, lotion, eye cream or gel, sun-screen, anti-wrinkle cream, face mask cream, lip gloss, lipstick, deodorant, aftershave, shampoo, cream rinse, anti-inflammatory medicine, anti-itching medicine and supplementary food.

9. The composition of claim 6, wherein said composition is included in a cosmetic.

10. The composition of claim 7, wherein said cosmetic comprises from about 0.01 % to about 20% w/w of said Mulberry extract.

11. The composition of claim 7, wherein said cosmetic comprises herbal plants and/or plant extracts.

12. The composition of claim 11, wherein said cosmetic is a cream or lotion and said herbal plants or plant extracts are *Pueraria mirifica, Glycine max, Vigna radiata, Sesamum indicum, Aloe vera, Clerodendrum inerme, Passiflora foetida, Mirabilis jalapa, Ardisia elliptica, Curcuma longa, Zingiber cassumunar, Curcuma xanthorrhiza, Stephania venosa, Gynura pseudochina, Centella asiatica, Phyllanthus emblica, Chromolaena odoratum, Pogostemon auricularis,* or tea tree oil.

13. The composition of claim 12, wherein said herbal plants or plant extracts comprise from about 0.5% to about 5.0% w/w of said cream or lotion.

14. The composition of claim 11, wherein said cosmetic is a soap or shampoo and said herbal plants or plant extracts are *Clitoria ternatea, Sapindus rarak, Sapindus emariginatus, Sapindus trifoliatus, Eclipta protata, Eclipta alba, Zingiber officinale, Hydnocarps anthelminthicus, Zollingeria dongnaiensis, Lawsonia inermis, Cymbopogon citratus, Citrus hystrix, Rhinacanthus nasutus, Andrographis paniculata, Tinospora crispa, Acacia concinna, Averrhoa carambola, Chromolaena odoratum, Pogostemon auricularis, Clitoria ternatea, Tamarindus indicus,* or tea tree oil.

15. The composition of claim 14, wherein said herbal plants or plant extracts comprises from about 0.5% to about 5.0% w/w of said soap or shampoo.

16. The composition of claim 1, wherein said Mulberry extract is taken from *Morus rotundiloba* Koidz, *Morus laevigata* Wall; or hybrids obtained from crosses between the former two plants and *Morus alba* Linne.

17. The composition of claim 2, wherein said organic solvent comprises ethyl alcohol, glycerine, sorbitol, propylyne glycol or butylene glycol, or combinations thereof.

18. The composition of claim 1, wherein said preservative comprises methyl paraben, propyl paraben, sodium benzoate, benzoic acid or a combination thereof.

19. The compostion of claim 3, wherein suspending agent comprises sorbitan sesquioleate, carbopol, emulgin, ceteareth-25, or a combination thereof.

20. The composition of claim 6, wherein said anti-oxidant comprises Vitamin C, ascorbic palmitate, tocoferol, Vitamin E or a combination thereof.

21. The composition of claim 1, wherein said Mulberry extract is obtained without an aging process and filtration of the resulting precipitate formed.
